# FASCICULE DE BREVET EUROPEEN

(11) **EP 3 516 367 B1**
(45) Date de publication et mention de la délivrance du brevet: **27.01.2021**
(21) Numéro de dépôt: 17778224.0
(22) Date de dépôt: 20.09.2017
(51) Int. Cl.: G01N 1/40, B01L 3/00, G01N 35/10

(54) **DISPOSITIF DE CAPTURE DE PARTICULES BIOLOGIQUES**
VORRICHTUNG ZUR ERFASSUNG BIOLOGISCHER PARTIKEL
DEVICE FOR CAPTURING BIOLOGICAL PARTICLES

(30) Priorité: 20.09.2016 FR 1658828
(43) Date de publication de la demande: 31.07.2019
(73) Titulaire: KABCYT, 92160 Antony (FR)
(72) Inventeur: KARKOUCHE, Bastien, 92160 Antony (FR)
(74) Mandataire: Nony
(86) Numéro de dépôt international: PCT/EP2017/073749
(87) Numéro de publication internationale: WO 2018/054961

(56) Documents cités:
- EP-A1- 1 972 945
- WO-A2-2010/012941
- DE-A1- 19 608 372
- US-A1- 2002 106 718
- US-A1- 2008 142 456

## Description

### Domaine technique

L'invention concerne le domaine de l'analyse de préparations biologiques à des fins de diagnostic médical. Plus précisément, elle se rapporte à un dispositif de capture de particules biologiques, notamment de cellules, en suspension dans un milieu liquide, et en particulier dans un échantillon biologique.

L'invention concerne également un procédé de capture de telles particules biologiques, ainsi qu'un appareil permettant la mise en œuvre de ce procédé.

### Etat de la technique

Un dispositif pour la capture de particules biologiques est décrit dans WO 2010/012941. Ce dispositif comporte un tube fermé par une membrane filtrante. Un bloc absorbant est placé à l'intérieur du tube. Lorsque le tube est plongé dans un milieu liquide, l'absorption d'eau par le bloc absorbant permet de contrôler le flux entrant dans le tube à travers la membrane filtrante. Des particules biologiques sont alors retenues sur la membrane filtrante.

La membrane filtrante est ensuite appliquée sur une lame et un flux sortant est produit à travers la membrane filtrante afin de reporter un échantillon de cellules, initialement retenues sur la surface extérieure de la membrane filtrante, sur ladite lame. La couche cellulaire reportée sur la lame permet avantageusement la réalisation d'une analyse cytologique fiable.

Une analyse cytologique permet notamment la détection de modifications à l'origine ou associées à des maladies pouvant affecter le pronostic vital, notamment la détection d'états cancéreux ou précancéreux comme les cancers du sein, des voies urinaires, de l'utérus, etc.

Le contrôle du flux entrant à travers la membrane filtrante permet de retenir un nombre de cellules suffisant pour obtenir un échantillon cellulaire statistiquement représentatif de la population cellulaire dans le milieu liquide. Il évite également d'obtenir, sur la membrane filtrante, un nombre de cellules trop élevé, ce qui conduirait à l'obtention d'un échantillon cellulaire dans lequel les cellules forment des amas et/ou des empilements, c'est-à-dire un échantillon à partir duquel l'analyse cytologique ultérieure ne serait pas optimale. En particulier, lorsque les cellules forment des amas et/ou des empilements, il existe un risque significatif que des cellules d'intérêt soient inaccessibles à l'analyse cytologique.

Il existe un besoin permanent pour améliorer encore la fiabilité de la capture des cellules.

Un but de l'invention est de répondre à ce besoin.

### Résumé de l'invention

Selon l'invention, on atteint ce but au moyen d'un dispositif de capture de particules biologiques en suspension dans un milieu liquide, le dispositif comportant :
- un récipient débouchant par une ouverture inférieure, de préférence un tube d'axe X débouchant, de préférence suivant l'axe X, à des extrémités inférieure et supérieure par des ouvertures inférieure et supérieure, respectivement ;
- une membrane filtrante fixée sur le récipient de manière à obturer l'ouverture inférieure ; et
à l'intérieur du récipient :
- un tampon en une mousse poreuse présentant une face plane reposant sur la membrane filtrante ;
- un bloc absorbant reposant sur le tampon et apte à absorber dudit milieu liquide lorsqu'il est en contact avec ledit milieu liquide, de préférence apte à gonfler sous l'effet d'un contact avec ledit milieu liquide, de préférence hydrophile; et
- un ressort entravant l'expansion et/ou le déplacement du bloc absorbant à l'écart de l'ouverture inférieure du récipient, et en particulier vers une extrémité supérieure du récipient, notamment lorsque le récipient est un dit tube.

De manière surprenante, l'inventeur a constaté que l'interposition d'un tel tampon entre le bloc absorbant et la membrane filtrante améliore remarquablement l'homogénéité de la couche de particules biologiques retenues sur ladite membrane filtrante. Le report de ces particules biologiques sur un substrat d'analyse, par exemple sur une lame, conduit avantageusement à un échantillon plus homogène, ce qui conduit à une analyse plus fiable.

Sans être lié par une théorie, l'inventeur explique ce résultat par la capacité de la mousse du tampon à se déformer pour compenser la déformation du bloc absorbant. Cette déformation ne modifie donc pas sensiblement la distribution de la pression exercée sur la face du tampon en appui sur la membrane filtrante, qui reste uniforme sur toute la zone d'appui du tampon sur la membrane filtrante.

Un dispositif de capture selon l'invention présente de préférence une ou plusieurs des caractéristiques optionnelles suivantes :
- l'épaisseur du tampon, mesurée le long de l'axe X, est supérieure à 1 mm et inférieure à 4 mm ;
- le matériau du tampon est de préférence du polyuréthane ;
- le tampon présente une face inférieure de forme complémentaire à la face supérieure de la membrane filtrante ;
- le tampon est en contact avec plus de 80% d'une face supérieure de la membrane filtrante ;
- le dispositif comporte de préférence une butée entravant le déplacement du ressort à l'écart de l'ouverture inférieure du récipient, et en particulier, lorsque le récipient est un dit tube, vers l'ouverture supérieure du tube ;
- le ressort est un bloc de mousse élastique ;
- le bloc de mousse est conformé de manière que, dans une position dans laquelle il est logé à l'intérieur du récipient, il soit compressé par la paroi latérale du récipient ;
- la taille moyenne des pores de la membrane filtrante est supérieure à un micron et/ou inférieure à 25 microns, et/ou le bloc absorbant est constitué en un matériau hydrophile ;
- le récipient est un tube d'axe X débouchant, à des extrémités inférieure et supérieure, par des ouvertures inférieure et supérieure, respectivement ;
- la différence entre les plus grandes dimensions transversales de l'ouverture supérieure du tube d'une part et du tampon d'autre part est supérieure à 0,2 mm et inférieure à 4 mm.

L'invention concerne aussi un procédé pour capturer des particules biologiques en suspension dans un milieu liquide au moyen d'un dispositif de capture selon l'invention, ledit procédé comportant les étapes suivantes :
i) immersion de la membrane filtrante du récipient dans le milieu liquide, de préférence en maintenant émergée l'extrémité supérieure dudit récipient ;
ii) maintien du récipient en position (dans la position au moins partiellement immergée obtenue à l'issue de l'étape i)), dans une position immobile ou, de préférence, oscillante autour de l'axe X lorsque le récipient est un dit tube, afin de mettre les particules biologiques en suspension homogène au sein du milieu liquide, pendant une durée suffisante pour retenir sur la membrane filtrante des particules contenues dans le flux de milieu liquide entrant dans le récipient et généré par l'absorption dudit milieu liquide par le bloc absorbant ;
iii) extraction du récipient hors du milieu liquide et, optionnellement, application de la membrane filtrante sur un substrat d'analyse.

L'invention concerne également un appareil d'analyse comportant :
- un portoir de flacons ;
- un support de récipients de dispositifs de capture selon l'invention;
- de préférence, un porte-doigts ;
- de préférence, un portoir de substrats d'analyse ;
- un mécanisme configuré pour
   - lorsque l'appareil d'analyse comporte un porte-doigts, introduire des doigts du porte-doigts dans des récipients de dispositifs de capture selon l'invention respectifs disposés sur le support de récipients, de manière à constituer un portoir de récipients,
   - introduire les membranes filtrantes des récipients disposés sur le support de récipients dans des flacons respectifs disposés sur le portoir de flacons, et pour extraire lesdits récipients hors desdits flacons respectifs, et
   - de préférence appliquer les membranes filtrantes des récipients sur des substrats d'analyse respectifs, de préférence disposés sur le portoir de substrats d'analyse.

Un appareil d'analyse selon l'invention présente de préférence une ou plusieurs des caractéristiques optionnelles suivantes :
- lorsque l'appareil d'analyse comporte un porte-doigts, chaque doigt est de préférence percé d'une lumière, ladite lumière débouchant par des ouvertures inférieure et supérieure en communication de fluide avec le volume intérieur d'un récipient respectif, après constitution du portoir de récipients, et avec le volume intérieur d'un soufflet, respectivement ;
- l'appareil d'analyse comportant un vérin configuré de manière à sélectivement presser ledit soufflet afin d'augmenter la pression à l'intérieur dudit récipient ;
- après constitution du portoir de récipients, chaque doigt présente une extrémité inférieure en contact avec un ressort d'un dit dispositif de capture.

L'invention concerne également un procédé de préparation d'un échantillon destiné à une analyse biologique, en particulier cytologique, ledit procédé comportant les étapes suivantes :
a) obtention d'au moins un flacon contenant un milieu liquide contenant des particules biologiques et, de préférence, disposition dudit flacon dans un portoir de flacons ;
b) de préférence, indépendamment de l'étape a), disposition d'un dispositif de capture selon l'invention sur un support de récipients ;
c) après l'étape b), lorsque l'appareil d'analyse comporte au moins un doigt, introduction dudit doigt, de préférence d'un doigt d'un porte-doigts, par une ouverture supérieure du récipient du dispositif, le doigt étant de préférence conformé de manière que, dans la position d'introduction maximale du doigt dans le récipient, le tampon contenu dans le récipient soit en contact sur la membrane filtrante fixée sur ledit récipient ;
d) introduction de la membrane filtrante du récipient dans ledit flacon et maintien du récipient en position pendant une durée suffisante pour que le bloc absorbant absorbe dudit milieu liquide entrant à l'intérieur du récipient à travers la membrane filtrante ;
e) extraction du récipient hors du flacon ;
f) récupération de particules biologiques retenues sur la membrane filtrante, de préférence par application de la membrane filtrante sur un substrat d'analyse après une période d'attente supérieure à 5 s, puis, de préférence, augmentation de la pression à l'intérieur du récipient de manière à créer un flux de milieu liquide sortant du récipient à travers la membrane filtrante.

Le procédé de préparation peut en particulier être mis en œuvre au moyen d'un appareil d'analyse selon l'invention.

### Définitions

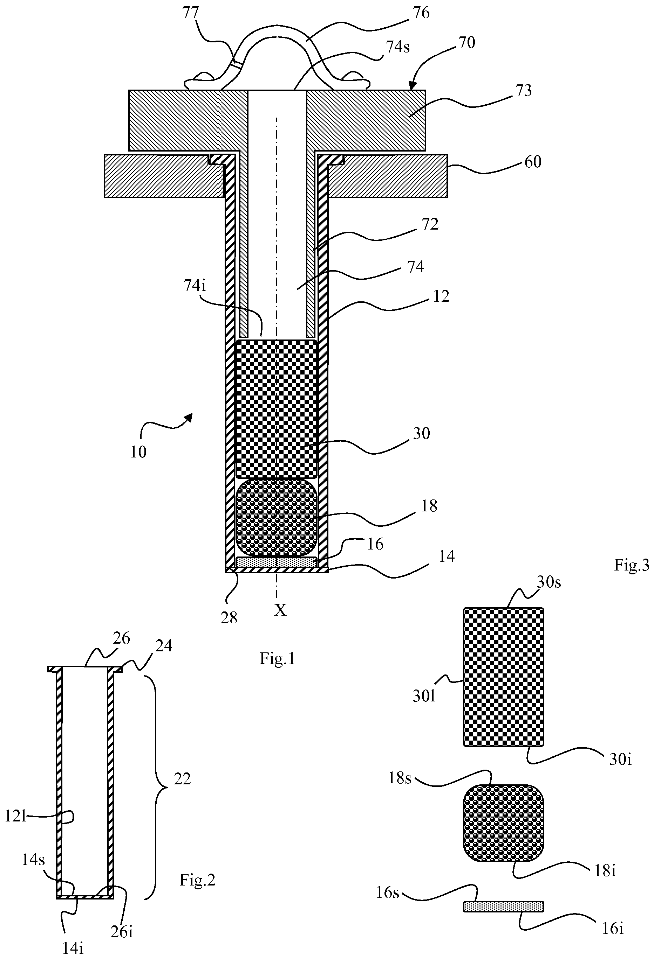

Dans le cadre général où le dispositif de capture comporte un « récipient », les adjectifs "supérieur" et "inférieur" ne sont pas limitatifs. Dans le cadre où le récipient est un tube d'axe X, ils sont définis en référence à une position d'un dispositif, dans laquelle l'axe X du tube est sensiblement vertical (comme sur la figure 1). Sauf indication contraire, "axial" fait référence à l'axe X du tube.

Sauf indication contraire, par "transversal", on entend une orientation perpendiculaire à l'axe X du tube.

Par "particules biologiques", on entend une particule non soluble dans un milieu liquide aqueux et susceptible d'être contenue dans un matériel biologique prélevé du corps d'un organisme vivant multicellulaire, animal ou végétal, en particulier un organisme vivant multicellulaire animal, notamment un mammifère, y compris l'homme. Les microfragments tissulaires, les micro-organismes, les cellules vivantes, les cellules mortes, les corps cellulaires anucléés tels que les érythrocytes et les plaquettes (thrombocytes), les fragments, les débris cellulaires, ainsi que les cristaux éventuels et les corps étrangers solides légers sont des exemples de particules biologiques. Les substances protéiques non solubles, telles que la pectine ou les substances protéiques dérivées de la fibronectine, par exemple les substances protéiques dérivées de la fibronectine fœtale, qui représentent un paramètre clinique indiquant un risque d'accouchement prématuré, sont d'autres exemples de particules biologiques.

La "capacité de gonflement" d'un bloc absorbant est le rapport entre le volume de ce bloc après gonflement maximal par absorption de milieu liquide et son volume initial à sec.

Sauf indication contraire, les verbes "comporter", "comprendre" ou "présenter" doivent être interprétés de manière large et non limitative.

### Brève description des figures

D'autres caractéristiques et avantages de l'invention apparaitront encore à la lecture de la description détaillée qui va suivre et à l'examen du dessin annexé dans lequel :
- la figure 1 représente un exemple d'un dispositif de capture selon l'invention, dans un plan de coupe longitudinale médian ;
- les figures 2 et 3 représentent des détails de la figure 1 ;
- la figure 4 (4a-4i) illustre schématiquement les différentes étapes d'un procédé de préparation selon l'invention, dans un mode de réalisation mettant en œuvre un appareil d'analyse selon l'invention ;
- les figures 5 et 6 représentent des deuxième et troisième modes de réalisation particuliers d'un dispositif de capture selon l'invention ; et
- la figure 7 illustre, schématiquement, un appareil selon l'invention.

### Description détaillée

La figure 1 représente un dispositif de capture 10 comportant un récipient sous la forme d'un tube 12 d'axe X, une membrane filtrante 14, un tampon 16, un bloc absorbant 18 et un ressort 20.

### Tube

Le tube 12 présente une portion cylindrique 22, de préférence de section transversale circulaire, terminée, à l'extrémité supérieure du tube 12, par un rebord 24, de préférence annulaire.

Dans un mode de réalisation préféré, le rebord 24 est interrompu, définissant ainsi des oreilles.

La longueur du tube est de préférence supérieure à 5 cm et/ou inférieure à 10 cm. La plus grande dimension transversale de la portion cylindrique 22 est de préférence supérieure à 1 cm et/ou inférieure à 4 cm.

L'épaisseur de la paroi latérale définissant la portion cylindrique 22 est de préférence supérieure à 1 mm et/ou inférieure à 5 mm, de préférence inférieure à 3 mm.

Le tube 12 débouche, à ses extrémités supérieure et inférieure, par des ouvertures supérieure et inférieure, référencées 26s et 26i, respectivement.

De préférence, le tube est en matière plastique, par exemple en chlorure de polyvynile, en polystyrène ou en polyéthylène.

Le tube 12 peut comporter une ou plusieurs des caractéristiques du tube référencé "101" décrit dans WO 2010/012941.

Le tube peut être du type de ceux couramment utilisés dans les systèmes automatisés de traitement d'échantillons biologiques pour l'analyse cytologique.

### Membrane filtrante

La membrane filtrante 14 est fixée sur le chant 28 du tube 12 qui définit l'ouverture inférieure 26i, de manière à obturer complètement ladite ouverture inférieure. De préférence, la membrane filtrante est collée, soudée par laser ou thermosoudée sur ledit chant.

Toutes les membranes filtrantes connues pour la filtration cellulaire dans le domaine de la cytologie, et en particulier les membranes filtrantes en polyester ou en polycarbonate, par exemple les membranes filtrantes commercialisées par la société MILLIPORE (BILLERICA, MA, Etats-Unis) ou par la société WHATAM GE HEALTHCARE (VERSAILLES, France), peuvent être utilisées.

Une membrane filtrante de la société IT4IP (Belgique) peut être également utilisée.

La taille moyenne des pores de la membrane filtrante est adaptée à l'application visée. De préférence, la taille moyenne des pores de la membrane filtrante est supérieure à un micron, supérieure à 3 µm, ou supérieure à 5 µm, et/ou inférieure à 200 microns, inférieure à 150 microns, inférieure à 100 microns, inférieure à 50 microns, ou inférieure à 25 microns.

Dans un mode de réalisation, elle est supérieure à 1,5 micron et/ou inférieure à 2,5 microns, de préférence d'environ 2 microns. La membrane filtrante peut être notamment la membrane référencée 7060-2511 commercialisée par la société WHATAM GE HEALTHCARE. Avantageusement, la totalité des particules biologiques d'intérêt pour une analyse cytologique peut être prélevée au moyen d'une telle membrane filtrante, quelle que soit la nature ou l'origine tissulaire du milieu liquide.

Dans un autre mode de réalisation, la membrane filtrante présente une taille moyenne de pores supérieure à 3 µm, de préférence supérieure à 5 µm et/ou inférieure à 10 µm, de préférence inférieure à 8 µm, de préférence inférieure à 7 µm. La membrane filtrante peut être alors en particulier une membrane filtrante référencée TMTT-02500 commercialisée par la société MILLIPORE ou référencée TTT-B02500 commercialisée par la société MILLIPORE, ou une membrane filtrante Cyclopore® PC telle que les membranes 5 µm référencées 7060-2513 ou 7060-4713, 8 µm, référencées 7060-2514 ou 7060-4714 ou 10 µm, référencées 7060-2515 ou 7060-4715. De telles membranes peuvent être en particulier utilisées pour ne retenir que des cellules de grandes tailles, par exemple du type des cellules épithéliales résultant d'un prélèvement ou d'un frottis cervico-vaginal.

### Tampon

Le tampon 16, aussi appelé « tampon adaptateur (TA) », est de préférence constitué en une mousse perméable au milieu liquide. Le milieu liquide entrant dans le tube à travers la membrane filtrante peut ainsi atteindre rapidement le bloc absorbant.

Le tampon 16 présente une porosité ouverte facilitant sa traversée par le milieu liquide.

De préférence, le tampon est configuré de manière que son volume reste sensiblement constant lorsqu'il est en contact avec de l'eau, et plus généralement avec le liquide ayant traversé la membrane filtrante.

De préférence, le tampon 16 présente une face inférieure 16i de forme sensiblement complémentaire à la face supérieure 14s de la membrane filtrante 14. De préférence, les faces 16i et 14s sont sensiblement planes, de préférence sensiblement transversales.

De préférence, le tampon 16 est en contact avec plus de 80%, de préférence plus de 90%, de préférence plus de 95%, de préférence sensiblement 100% de la face supérieure 14s de la membrane filtrante 14 (exposée vers l'intérieur du tube 12).

De préférence, le tampon 16 présente une forme cylindrique d'axe X. De préférence, la surface latérale du tampon 16 est sensiblement complémentaire à la surface intérieure 12l du tube 12.

De préférence, le tampon présente des dimensions adaptées pour qu'il puisse être déplacé librement dans le tube. L'assemblage du dispositif en est facilité.

Avantageusement, lors de l'assemblage, le tampon, introduit par l'ouverture supérieure du tube 12, peut être ainsi glissé, par gravité, le long du tube, jusqu'à entrer en butée avec la membrane filtrante.

De préférence, la différence entre les plus grandes dimensions transversales de l'ouverture supérieure du tube d'une part et du tampon d'autre part est supérieure à 0,2 mm, supérieure à 0,5 mm, de préférence supérieure à 0,8 mm et/ou inférieure à 4 mm, de préférence inférieure à 3 mm, de préférence inférieure à 2,5 mm.

L'épaisseur du tampon, mesurée le long de l'axe X, est de préférence constante, et de préférence supérieure à 1 mm, de préférence supérieure à 1,5 mm et/ou de préférence inférieure à 4 mm, de préférence inférieure à 3 mm, de préférence inférieure à 2,5 mm. Les meilleurs résultats ont été obtenus avec une épaisseur de 2 mm.

### Bloc absorbant

Le bloc absorbant 18 peut présenter une ou plusieurs des caractéristiques du bloc de matériau absorbant décrit dans WO 2010/012941.

De préférence, le bloc absorbant 18 présente une forme cylindrique d'axe X, de préférence de section circulaire, de préférence sensiblement complémentaire à la surface intérieure 12l du tube 12. De préférence, il présente des dimensions adaptées pour qu'il puisse être déplacé sensiblement librement à l'intérieur du tube. L'assemblage du dispositif en est facilité.

Avantageusement, lors de l'assemblage, le bloc absorbant, introduit par l'ouverture supérieure du tube 12, peut être ainsi glissé, par gravité, le long du tube, jusqu'à entrer en butée avec le tampon 16.

De préférence, la différence entre les plus grandes dimensions transversales de l'ouverture supérieure du tube d'une part et du bloc absorbant d'autre part est supérieure à 0,2 mm, supérieure à 0,5 mm, de préférence supérieure à 0,8 mm et/ou inférieure à 4 mm, de préférence inférieure à 3 mm, de préférence inférieure à 2,5 mm.

L'épaisseur du bloc absorbant, mesurée selon la direction de l'axe X, est de préférence supérieure à 5 mm, supérieure à 8 mm, supérieure à 9 mm et/ou inférieure à 30 mm, inférieure à 20 mm, de préférence inférieure à 15 mm, de préférence inférieure à 12 mm.

De préférence, le bloc absorbant 18 présente une face inférieure 18i de forme sensiblement complémentaire à la face supérieure 16s du tampon 16. De préférence, les faces 16s et 18i sont sensiblement planes, de préférence sensiblement transversales.

Le bloc absorbant 18 comporte, de préférence est constitué en un matériau qui gonfle sous l'effet d'un contact avec un milieu liquide.

De préférence, le bloc absorbant est constitué en un matériau hydrophile qui gonfle lorsque mis en contact avec un milieu liquide aqueux, en particulier avec de l'eau. De préférence, ce matériau comporte, de préférence est constitué de viscose, de préférence de viscose compressée. Dans un mode de réalisation préféré, le bloc absorbant 18 est constitué d'un empilement de feuilles de viscose, de préférence de feuilles de viscose non tissées, l'empilement desdites feuilles ayant été compressé. La viscose présente l'avantage d'une bonne capacité d'absorption d'eau, mais aussi d'une bonne capacité de gonflement sous l'effet de cette absorption.

De préférence, le bloc absorbant présente une capacité de gonflement supérieure à 2, de préférence supérieure à 3, de préférence supérieure à 4. Une telle capacité de gonflement peut être notamment obtenue avec de la viscose.

Le bloc absorbant 18 peut également comporter, voire être constitué, d'un agent super absorbant, bien connu de l'homme du métier, par exemple du type hydrogel. L'agent super absorbant peut être en particulier un polymère du type polyacrylate de sodium réticulé, qui peut être obtenu par une réaction de polymérisation d'un acide acrylique mélangé à de l'hydroxyde de sodium en présence d'un agent initiateur de polymérisation, un copolymère de poly-acrylamide, un copolymère d'anhydride maléique d'éthylène, une carboxyméthyl cellulose réticulée, un copolymère d'alcool polyvinylique ou un oxyde de polyéthylène réticulé.

Dans un mode réalisation, la capacité de gonflement est supérieure à 10, supérieure à 15, de préférence supérieure à 20, voire supérieure à 30. Une telle capacité de gonflement est notamment possible avec du polyacrylate de sodium réticulé, dont la capacité de gonflement peut atteindre 60.

### Ressort

Le ressort 20, ou « tampon ressort (TR) » a pour fonction
- d'entraver, sans bloquer, l'expansion du bloc absorbant et son déplacement vers l'ouverture supérieure du tube, ce qui limite la déformation de la membrane filtrante, et
- de maintenir en contact permanent le bloc absorbant et le tampon, mais aussi le tampon et la membrane filtrante.

L'élasticité du ressort est de préférence suffisante pour compenser un allongement du bloc absorbant, selon l'axe X, de plus de 5%, de préférence de plus de 10%, voire de plus de 15%.

Dans un mode de réalisation préféré, le ressort 20 comporte un bloc de mousse 30 disposé sur la face supérieure 18s du bloc absorbant 18.

Le bloc de mousse peut présenter la forme d'un parallélépipède rectangle, par exemple d'un cube, dont la plus grande dimension peut être supérieure à 1 cm, supérieure à 1,5 cm, supérieure à 2 cm et/ou inférieure à 4 cm, ou inférieure à 3 cm.

L'élasticité du bloc de mousse est de préférence telle qu'elle permet d'en réduire le volume d'un facteur supérieur à 2, de préférence supérieur à 4, de préférence supérieur à 6, de préférence supérieur à 8, de préférence supérieur à 10, de préférence supérieur à 15, de préférence supérieur à 20, par compression manuelle, par exemple entre l'index et le pouce.

L'élasticité et le volume du bloc de mousse sont de préférence déterminés pour que la déformation du bloc de mousse puisse compenser élastiquement l'augmentation de volume du bloc absorbant lors de son gonflement.

La mousse du bloc de mousse 30 peut être à base de polyols et d'isocyanates. Il peut être en en polyuréthane. Il peut être en particulier en RICHLUX HIGH RESILIENCE HR50065, RG50030 ou RG50036, commercialisé par la société CARPENTER BELGIUM NV (Belgique).

Pour que le ressort, et en particulier la mousse, puisse exercer son effet de ressort, son déplacement vers l'ouverture supérieure du tube doit être entravé, voire bloqué. Comme on le verra plus en détail, cette entrave du ressort peut notamment résulter de l'appui d'un doigt introduit dans le tube par son ouverture supérieure, ou de l'appui du ressort sur une butée du tube.

De préférence, le bloc de mousse s'étend, à l'intérieur du tube, sur une hauteur, mesurée le long de l'axe X, inférieure à 4 cm, de préférence inférieure à 3 cm, de préférence inférieure à 2,5 cm.

Dans un mode de réalisation, le bloc de mousse est conformé de manière à frotter sur la surface intérieure 12l de la paroi latérale du tube. Dans un mode de réalisation, la plus grande dimension dans une coupe transversale du bloc de mousse, mesurée alors que le bloc de mousse est sorti du tube, est légèrement supérieure au diamètre intérieur du tube, par exemple supérieure de 0,2 mm, supérieure de 0,5 mm, ou supérieure de 1 mm audit diamètre. Pour être introduit dans le tube, le bloc de mousse doit donc être légèrement comprimé latéralement. Avantageusement, la nécessité de devoir comprimer latéralement le bloc de mousse pour l'introduire dans le tube facilite le maintien, dans le tube, du tampon 16 et du bloc absorbant 18.

Avantageusement, le frottement du bloc de mousse entrave son déplacement lors de l'expansion du bloc absorbant.

Dans un mode de réalisation, pour entraver le déplacement du bloc de mousse 30, en complément ou en remplacement dudit frottement, une butée est disposée au-dessus du bloc de mousse 30. Cette butée peut être intégrée dans le dispositif ou être rapportée au moment où le dispositif doit être utilisé, comme le doigt décrit ci-après.

Dans un mode de réalisation préféré, le bloc de mousse 30 présente une face inférieure 30i sensiblement complémentaire à la face supérieure 18s du bloc absorbant, de préférence sensiblement plane, de préférence sensiblement transversale.

La forme du bloc de mousse 30 n'est pas limitative. En particulier, l'aire de la surface latérale 30l du bloc de mousse 30 qui est en contact avec la surface intérieure du tube 12 n'est pas limitative et peut être avantageusement modifiée en fonction de l'effet de ressort souhaité.

Le ressort 20 peut également comprendre, voire être constitué par d'autres moyens élastiques qu'un bloc de mousse, par exemple peut comporter un ressort hélicoïdal dont une extrémité inférieure est en appui sur le bloc absorbant 18 et l'extrémité opposée est immobilisée par rapport au tube, au moins provisoirement, par exemple par une butée fixée sur le tube.

### Appareil d'analyse

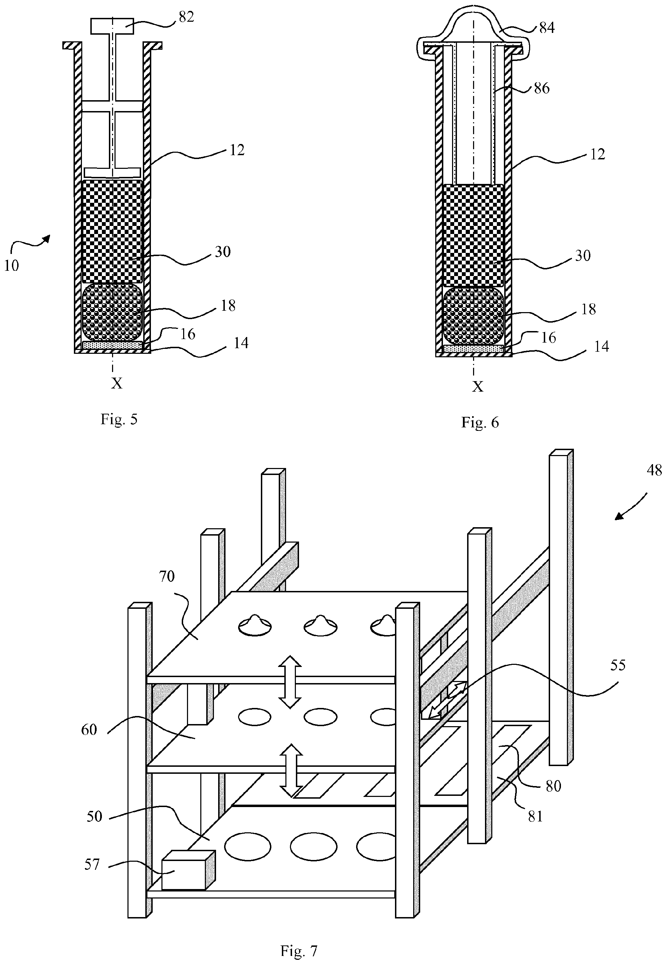

Un dispositif de capture peut être avantageusement utilisé avec un appareil d'analyse 48 selon l'invention, illustré sur la figure 7. Un appareil d'analyse 48 comporte un portoir de flacons 50, un portoir de récipients, en l'occurrence un portoir de tubes 52, constitué par assemblage d'un support de récipients, en l'occurrence un support de tubes 60, et d'un porte-doigts 70 optionnel, optionnellement un portoir de substrat d'analyse 81 et un mécanisme 55 pour déplacer ces différents éléments les uns par rapport aux autres pour exécuter les étapes a) à f).

Le mécanisme 55 de l'appareil d'analyse 48 n'est pas limitatif, pourvu qu'il permette de déplacer, les uns par rapport aux autres, le portoir de flacons 50, le support de tubes 60, le porte-doigts 70, puis le portoir de tubes 52 résultant de l'assemblage du support de tubes et du porte-doigts 70, et le portoir de substrats d'analyse 81. La réalisation d'un tel mécanisme ne pose pas de difficultés particulières.

De préférence, un appareil selon l'invention comporte encore un automate 57 adapté pour commander le mécanisme 55 afin que les différentes étapes du procédé puissent s'enchaîner sans intervention humaine.

Le portoir de flacons 50 présente de préférence la forme d'une plaque percée d'un ou plusieurs orifices pour flacon 54 configurés pour recevoir chacun un flacon 56 contenant du milieu liquide chargé de particules biologiques à capturer.

Le portoir de flacons 50 comporte de préférence plus d'un, de préférence plus de deux, de préférence plus de cinq, de préférence plus de dix orifices pour flacon 54 adaptés pour retenir chacun un flacon 56 dans une position sensiblement verticale. A cet effet, le flacon 56 comporte de préférence un rebord annulaire qui l'empêche de traverser l'orifice pour flacon 54 dans lequel il est disposé.

Le portoir de tubes 52 comporte de préférence un support de tubes 60, de préférence sous la forme d'une plaque percée d'un ou plusieurs orifices pour tube 62 configurés pour recevoir chacun un dispositif de capture selon l'invention. En particulier, chaque orifice pour tube 62 peut être conformé de manière à laisser passer la portion cylindrique 22 du tube 12 d'un dispositif, tout en empêchant le passage du rebord 24.

Le nombre d'orifices pour tube 62 du support de tubes 60 est de préférence identique au nombre d'orifices pour flacon 54 du portoir de flacons.

De préférence, le portoir de tubes 52 comporte encore des moyens pour immobiliser chaque dispositif de capture sur le support de tubes 60, par exemple des mâchoires solidaires du support de tubes et venant serrer les tubes suspendus dans leurs orifices de tube respectifs.

Le porte-doigts 70 présente un ou plusieurs doigts 72 ou « poussoirs » aptes à être introduits dans des tubes disposés sur le portoir de tubes.

De préférence, chaque doigt présente, extérieurement, la forme générale d'une tige sensiblement rectiligne.

La longueur d'un doigt est de préférence supérieure à 1 cm, à 2 cm, à 5 cm.

Les doigts font saillie d'une base 73, par exemple sous la forme d'une plaque, à laquelle ils sont de préférence rigidement fixés. L'introduction de tous les doigts dans les tubes respectifs, à l'étape c), peut être ainsi avantageusement simultanée.

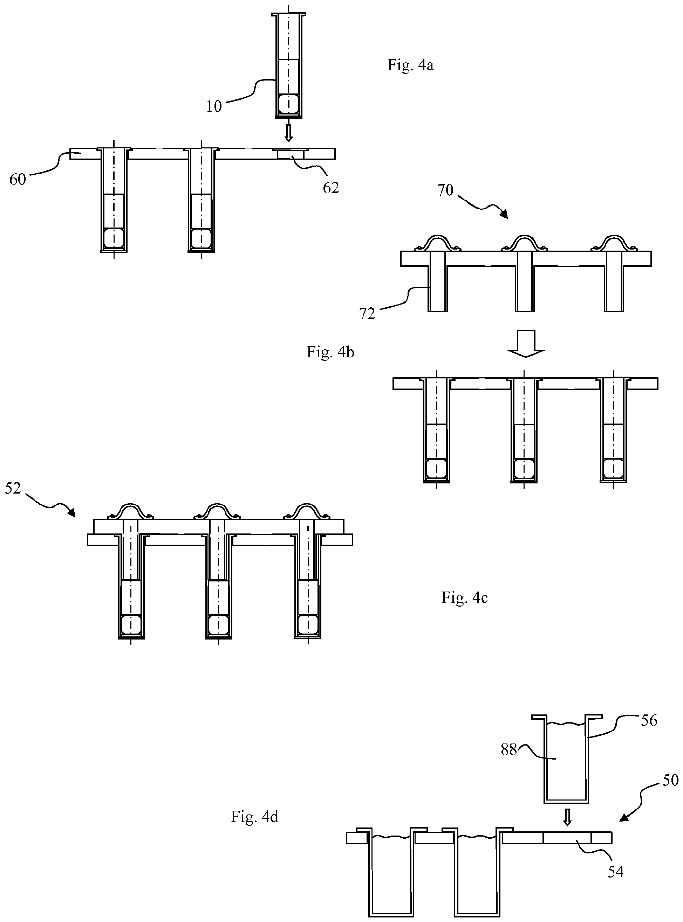

Le porte-doigts 70 comporte autant de doigts qu'il y a d'orifices pour tube 62 sur le support de tubes 60. Après introduction d'un doigt dans un tube, comme représenté sur la figure 4c, le doigt s'étend sensiblement selon l'axe X.

De préférence, chaque doigt 72 est percé d'une lumière longitudinale 74 débouchant par des ouvertures inférieure 74i et supérieure 74s vers le bloc de mousse 30 et dans le volume intérieur d'un soufflet 76. L'actionnement du soufflet, c'est-à-dire sa compression de manière à en réduire le volume permet ainsi d'insuffler le gaz contenu dans le soufflet, de préférence de l'air, à l'intérieur de la lumière longitudinale 74, et donc sur le bloc de mousse 30.

En position d'introduction maximale d'un doigt 72 dans un tube 12, comme représenté sur la figure 1, l'extrémité inférieure du doigt est de préférence en contact avec la face supérieure 30s du bloc de mousse 30, dans une position qui garantit que le tampon est en contact avec la membrane filtrante.

Avant utilisation du dispositif de capture, les différents composants à l'intérieur du tube ont en effet pu se déplacer, par exemple pendant leur transport. Si ces composants ne sont plus dans la position de service représentée sur la figure 1, l'introduction du doigt les repoussent vers la membrane filtrante jusqu'à positionner le bloc de mousse en contact avec le bloc absorbant, le bloc absorbant en contact avec le tampon, et le tampon en contact avec la membrane filtrante (figure 1).

Avantageusement, le doigt 72 peut également servir de butée pour limiter le déplacement du bloc de mousse 30 vers l'ouverture supérieure du tube lors de l'expansion du bloc absorbant.

De préférence, le soufflet 76 est pourvu d'un évent 77 autorisant une évacuation d'air limitée lorsqu'une pression est exercée sur le soufflet 76 afin d'en réduire le volume intérieur. L'évent 77 permet également à l'intérieur du tube 12 d'être toujours à la pression atmosphérique pendant le gonflement du bloc absorbant 18.

Le soufflet, qui présente de préférence la forme d'une tétine ou d'une ventouse, est de préférence fixé sur le porte-doigts, et de préférence sur la surface supérieure du porte-doigts 70, par exemple au moyen de rivets.

### Fonctionnement

Le fonctionnement du dispositif de capture selon l'invention est décrit dans le cadre du procédé de préparation selon l'invention.

**L'étape a)** est illustrée sur la figure 4d.

Le flacon 56 peut être en particulier un flacon couramment utilisé pour le conditionnement des prélèvements de cellules ou de tissus à des fins d'analyses biologiques, y compris d'analyses cytologiques ou histologiques.

Le milieu liquide 88 contenu dans le flacon peut consister en un milieu liquide aqueux tamponné contenant un agent de fixation des cellules ou des corps cellulaires en suspension. En tant qu'agent fixateur, on peut citer notamment les mélanges à base d'alcool, par exemple l'agent commercialisé sous la marque SEDFIX® par la société SURGIPATH ou celui commercialisé sous la marque PRESERVCYT® par la société HOLOGIC ou commercialisé sous la marque EASYFIX® par la société VWR. En particulier, lorsque l'analyse cytologique doit être réalisée à partir de cellules vivantes, le milieu liquide peut consister en un milieu tampon salin, de préférence en un milieu de culture cellulaire approprié. Le milieu liquide peut encore consister en un fluide corporel naturel tel que le sang, l'urine, ou toute sécrétion physiologique naturelle ou pathologique comme une ascite, un épanchement, un kyste ou encore un écoulement.

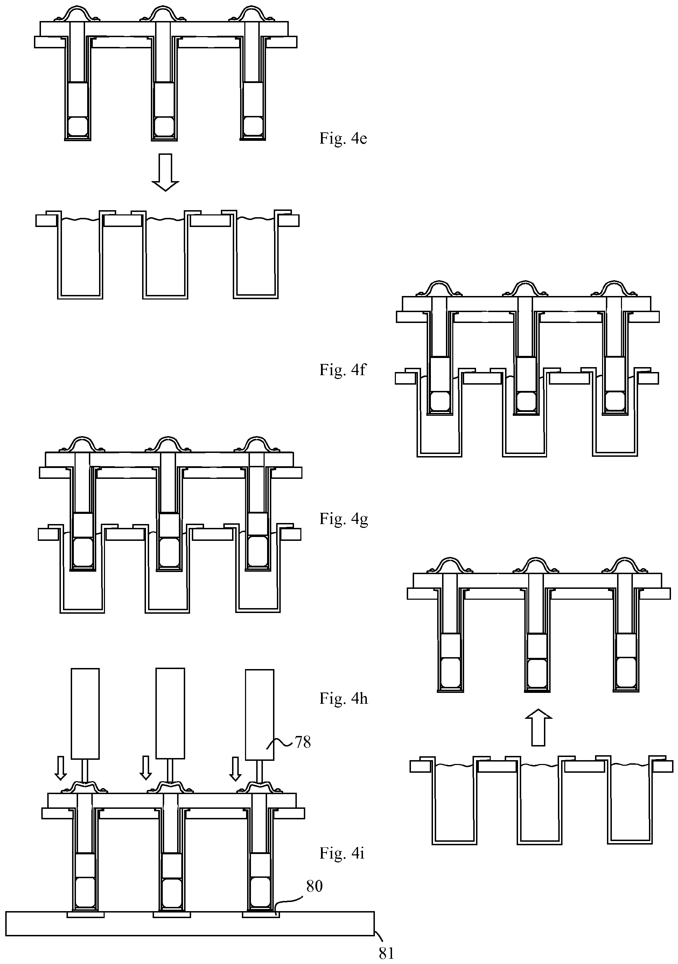

**A l'étape b),** les tubes d'un ensemble de dispositifs de capture 10 selon l'invention sont disposés dans les orifices pour tube 62 d'un support de tubes 60, comme illustré sur la figure 4a. Les dispositifs de capture sont au moins disposés dans les orifices pour tube 62 qui, lors du rapprochement du portoir de tubes et du portoir de flacons, seront en regard des flacons disposés dans les orifices pour flacon, comme représenté sur la figure 4e.

**A l'étape c),** les doigts sont disposés en regard des orifices pour tube 62 de manière à pouvoir être introduits dans les tubes des dispositifs de capture disposés dans les orifices pour tube 62, comme représenté sur la figure 4b.

Les doigts 72 sont ensuite introduits chacun dans un tube correspondant (figure 4b) jusqu'à ce que le porte-doigts 70 entre en butée sur le support de tubes 60. L'ensemble constitué par le support de tubes 60 et le porte-doigts 70 constitue un portoir de tubes 52 qui, de préférence, n'est plus désolidarisé jusqu'à la fin de la dernière étape du procédé (figure 4c).

**A l'étape d),** le portoir de tubes est disposé de manière que les tubes qu'il porte soient en regard des ouvertures supérieures des flacons 56 afin de pouvoir être introduits dans lesdits flacons (figure 4e). La profondeur de pénétration des tubes dans les flacons est déterminée de manière que la membrane filtrante 14, le tampon 16 et au moins une partie du bloc filtrant 18 de chaque tube soient sous la surface supérieure du milieu liquide contenu dans les flacons 56. Par pression hydrostatique, du milieu liquide pénètre donc à l'intérieur du tube, à travers la paroi filtrante 14, puis mouille le bloc absorbant 18.

L'appareil représenté est prévu pour le traitement simultané de trois flacons de prélèvement. Le fonctionnement de l'appareil est cependant le même quel que soit le flacon considéré. Dans la suite de la description, ce fonctionnement n'est donc décrit que pour un seul flacon.

Comme l'illustrent les figures 4f et 4g, le bloc filtrant 18 contenu dans le tube introduit dans ce flacon absorbe une partie du milieu liquide ayant pénétré dans le tube 12 et gonfle. En particulier, le gonflement du bloc absorbant 18 conduit à son expansion vers l'ouverture supérieure du tube, à l'encontre du bloc de mousse 30, et vers la membrane filtrante 14, à l'encontre du tampon 16 et de la membrane filtrante 14. La présence du bloc absorbant assure un flux entrant minimal, notamment par la force de tension superficielle résultant des caractéristiques d'énergie de surface du bloc absorbant et de l'action mécanique d'aspiration résultant de l'expansion du bloc absorbant.

Le doigt 72 s'oppose au déplacement du bloc de mousse 30 vers l'ouverture supérieure du tube et contraint donc ce dernier à se contracter. L'élasticité du bloc de mousse 30 permet avantageusement de maintenir le bloc absorbant 18 contre le tampon 16 et le tampon 16 contre la membrane filtrante 14.

Le tampon 16 permet une bonne répartition de la pression exercée par le bloc absorbant 18 et assure un flux entrant sensiblement homogène à travers la membrane filtrante 14.

Lors du passage du milieu liquide à travers la membrane filtrante 14, les particules biologiques sont retenues sur la face inférieure 14i de la membrane filtrante 14. L'uniformité du flux du milieu liquide à travers la membrane filtrante assure avantageusement une distribution homogène des particules biologiques sur la face inférieure 14i.

Le tube est maintenu dans le flacon pendant une durée déterminée en fonction de la quantité de particules biologiques à fixer sur la membrane 14 filtrante (figure 4g). Le maintien dans la position partiellement immergée dure de préférence plus de 5 secondes et/ou moins de 10 minutes. De préférence, cette durée est adaptée à la nature du milieu liquide, et en particulier à la concentration des particules biologiques dans le milieu liquide, à la densité de particules biologiques souhaitée sur la membrane filtrante et à la capacité d'absorption du bloc absorbant.

Le portoir de tubes est ensuite écarté du portoir de flacons, comme représenté sur la figure 4h, de manière à extraire complètement le tube hors du flacon.

Les particules biologiques sont alors récupérées pour constituer un échantillon adapté pour être observé, notamment afin de réaliser une analyse cytologique.

Dans un mode de réalisation préféré, la surface inférieure 14i de la membrane filtrante est appliquée sur un substrat d'analyse 80, comme représenté sur la figure 4i.

Préalablement à cette application, le tube est de préférence maintenu hors du flacon et sans contact avec le substrat d'analyse pendant une période d'attente de préférence supérieure à 5 s, de préférence supérieure à 30 s, de préférence supérieure à 1 minute, et de préférence inférieure à 5 minutes, de préférence inférieure à 3 minutes, de préférence inférieure à 2 minutes. Cette période d'attente est de préférence déterminée pour que le bloc absorbant absorbe le milieu liquide résiduel présent dans le tube et qui est « libre », c'est-à-dire qui n'est pas contenu dans le tampon ni dans le bloc absorbant.

Le substrat d'analyse 80 peut être en particulier une lame porte-objets.

De préférence, le substrat d'analyse 80 est disposé sur un portoir de substrats 81, le portoir de substrats étant de préférence configuré pour recevoir autant de substrats d'analyse que le support de tubes 60 peut recevoir de tubes.

De préférence, l'appareil d'analyse comporte des moyens pour générer un flux de milieu liquide sortant des tubes quand les membranes filtrantes sont appliquées sur des substrats d'analyse respectifs.

Plus précisément, une pression est exercée sur le soufflet 76 (flèches sur la figure 4i) afin d'exercer une surpression d'une courte durée. De préférence, la déformation du soufflet est effectuée au moyen d'un actionneur 78 sous la forme d'un vérin dont la tige vient appuyer rapidement sur ledit soufflet. Cette surpression crée un flux de milieu liquide hors du tube, à travers la membrane filtrante 14, ce qui conduit à décoller les particules biologiques de cette membrane. Les particules biologiques ainsi décollées sont ainsi reportées sur le substrat d'analyse 80.

De préférence, lorsque la tige du vérin se rétracte, le soufflet reprend élastiquement sa forme initiale.

Ce procédé de transfert des particules biologiques par réplique de la membrane filtrante est une méthode classiquement utilisée par les anatomopathologistes.

D'autres procédés de transfert sont possibles. En particulier, la membrane filtrante peut être simplement pressée contre le substrat d'analyse.

L'échantillon peut être ensuite soumis à une ou plusieurs étapes de traitement préalable à son observation, par exemple une ou plusieurs étapes de coloration spécifique ou non spécifique, y compris des étapes de coloration MAY-GRÜMWALD GIESMA, coloration dite "Papanicolaou", coloration de Shorr, hématoxyline, éosine, etc.

Préalablement à leur observation, les particules biologiques transférées peuvent également subir une étape d'incubation en présence d'anticorps détectables spécifiques de marqueurs membranaires ou de marqueurs intracellulaires, et/ou un traitement par une technique de biochimie moléculaire, par exemple par une technique d'hybridation *in situ* avec des sondes nucléiques spécifiques ou une technique d'extraction d'ARN et de quantification du niveau d'expression d'un ou plusieurs gènes d'intérêt, ou une technique d'extraction d'ADN et de détection de mutation dans la séquence d'un ou plusieurs gènes d'intérêt.

Dans un mode de réalisation, la membrane filtrante 14 est désolidarisée et l'ensemble constitué par la membrane filtrante et les particules biologiques retenues sont incluses dans de la paraffine ou une résine adaptée, ce qui est particulièrement utile pour récupérer des microfragments tissulaires à des fins d'analyse, en particulier pour la réalisation de coupes histologiques.

Le mode de réalisation du dispositif de capture illustré sur les figures 1 et 4 est particulièrement bien adapté à une automatisation.

L'invention n'est cependant pas limitée à ce mode de réalisation.

En particulier, comme représenté sur la figure 5, le dispositif de capture peut comprendre un piston 82, qui remplace le doigt 72 et l'actionneur 78. Toutes les étapes peuvent être réalisées manuellement, le piston étant activable à la main.

Ainsi, dans le mode de réalisation de la figure 5, le dispositif de capture comporte-t-il avantageusement les moyens pour décoller manuellement les particules biologiques de la membrane filtrante 14.

Le piston 82 peut en particulier comporter une ou plusieurs des caractéristiques optionnelles du piston "104" décrit dans WO 2010/012941. Comme décrit dans WO 2010/012941, l'ouverture supérieure du tube 12 peut être également fermée par un bouchon comportant un orifice guidant le coulissement du piston 82 dans le tube.

Dans le mode de réalisation de la figure 6, le piston est remplacé par un soufflet 84, similaire au soufflet 76, et qui fonctionne de manière identique. Le soufflet 84 est cependant fixé sur le bord supérieur du tube.

Par ailleurs, une butée est prévue pour bloquer le bloc de mousse 30. La forme de cette butée n'est pas limitative. Elle peut être par exemple constituée par un tube intérieur 86 logé dans le tube 12, similaire au doigt 72, de préférence immobilisé par rapport au tube. Un rebord supérieur 88 du tube intérieur 86 peut être par exemple pris en sandwich entre le soufflet 84 et le tube 12, comme représenté.

Le dispositif de capture est ainsi autonome et peut être utilisé à la main.

Comme cela apparaît clairement à présent, l'invention fournit un dispositif de capture favorisant, grâce à la présence du tampon 16, une répartition homogène des particules biologiques retenues sur la membrane filtrante 14. La fiabilité des analyses effectuées à partir de ces particules biologiques en est améliorée.

Par ailleurs, l'invention fournit un appareil d'analyse permettant une automatisation des différentes étapes du procédé de préparation d'un échantillon observable, en particulier pour une analyse cytologique. Cet appareil permet avantageusement de multiplier les mesures effectuées.

Bien entendu, l'invention n'est pas limitée aux modes de réalisation décrits en détail et/ou illustrés, fournis à des fins illustratives seulement. En particulier, le récipient n'est pas limité à un tube. Toutes les caractéristiques décrites ci-dessus pour un tube peuvent donc être appliquées à une autre forme de récipient, hormis celles qui sont spécifiques à une forme tubulaire. La membrane filtrante n'est pas nécessairement fixée à une extrémité inférieure du récipient, même si cela est préféré.

Le récipient peut présenter des dimensions quelconques et une structure quelconque, pourvu qu'il soit conformé pour que le ressort entrave l'expansion et/ou le déplacement du bloc absorbant.

Dans un mode de réalisation, le ressort peut être intégré dans la paroi du récipient, la paroi du récipient étant de préférence déformable élastiquement pour entraver l'expansion et/ou le déplacement du bloc absorbant. Par exemple, tout ou partie de la paroi du récipient peut être constituée par un film déformable élastiquement et tendu de manière à exercer un appui élastique sur le bloc absorbant.

Dans un mode de réalisation, le récipient ne comporte pas d'ouverture supérieure. La paroi du récipient définit alors avec la membrane filtrante une chambre fermée, ne communiquant de préférence avec l'extérieur que par l'intermédiaire de la membrane filtrante.

La paroi du récipient peut être rigide ou souple.

Enfin, l'invention n'est pas limitée à un domaine d'application particulier. Elle peut être par exemple également être utilisée pour la recherche de la légionellose.

## Revendications

1. Dispositif de capture de particules biologiques en suspension dans un milieu liquide, le dispositif comportant :
- un récipient (12) débouchant par une ouverture inférieure ;
- une membrane filtrante (14) fixée sur le récipient de manière à obturer l'ouverture inférieure ; et
à l'intérieur du récipient :
- un tampon (16) en une mousse poreuse présentant une face plane reposant sur la membrane filtrante ;
- un bloc absorbant (18) reposant sur le tampon et apte à absorber dudit milieu liquide lorsqu'il est en contact avec ledit milieu liquide; et
- un ressort (30) agencé pour entraver l'expansion et/ou le déplacement du bloc absorbant à l'écart de l'ouverture inférieure du récipient.

2. Dispositif selon la revendication immédiatement précédente, dans lequel l'épaisseur du tampon, mesurée le long de l'axe X, est supérieure à 1 mm et inférieure à 4 mm.

3. Dispositif selon l'une quelconque des revendications précédentes, dans lequel le tampon est en polyuréthane.

4. Dispositif selon l'une quelconque des revendications précédentes, dans lequel le tampon (16) présente une face inférieure (16i) de forme complémentaire à la face supérieure (14s) de la membrane filtrante (14).

5. Dispositif selon l'une quelconque des revendications précédentes, dans lequel le tampon (16) est en contact avec plus de 80% d'une face supérieure (14s) de la membrane filtrante (14).

6. Dispositif selon l'une quelconque des revendications précédentes, comportant une butée (72) entravant le déplacement du ressort à l'écart de l'ouverture inférieure du récipient.

7. Dispositif selon l'une quelconque des revendications précédentes, dans lequel le ressort est un bloc de mousse élastique.

8. Dispositif selon l'une quelconque des revendications précédentes, dans lequel le bloc de mousse est conformé de manière que, dans une position dans laquelle il est logé à l'intérieur du récipient, il soit compressé par la paroi du récipient.

9. Dispositif selon l'une quelconque des revendications précédentes, dans lequel la taille moyenne des pores de la membrane filtrante est supérieure à un micron et/ou inférieure à 25 microns, et/ou le bloc absorbant est constitué en un matériau hydrophile.

10. Dispositif selon l'une quelconque des revendications précédentes, dans lequel le récipient est un tube d'axe X débouchant par des extrémités inférieure et supérieure par des ouvertures inférieure (26i) et supérieure (26s), respectivement, le ressort étant apte à entraver l'expansion et/ou le déplacement du bloc absorbant vers l'extrémité supérieure du tube.

11. Dispositif selon la revendication immédiatement précédente, dans lequel la différence entre les plus grandes dimensions transversales de l'ouverture supérieure du tube d'une part et du tampon d'autre part est supérieure à 0,2 mm et inférieure à 4 mm.

12. Procédé pour capturer des particules biologiques en suspension dans un milieu liquide au moyen d'un dispositif de capture selon l'une quelconque des revendications précédentes, ledit procédé comportant les étapes suivantes :
i) immersion, dans le milieu liquide, de la membrane filtrante du récipient dudit dispositif de capture ;
ii) maintien du récipient en position pendant une durée suffisante pour retenir, sur la membrane filtrante dudit dispositif de capture, des particules contenues dans un flux de milieu liquide entrant dans le récipient et généré par l'absorption dudit milieu liquide par le bloc absorbant dudit dispositif de capture;
iii) extraction du récipient hors du milieu liquide et, optionnellement, application de la membrane filtrante sur un substrat d'analyse.

13. Procédé selon la revendication immédiatement précédente, dans lequel le récipient est un tube d'axe X débouchant par des extrémités inférieure et supérieure par des ouvertures inférieure (26i) et supérieure (26s), respectivement, et dans lequel, à l'étape i), l'extrémité inférieure du tube est immergée dans le milieu liquide, en maintenant émergée l'extrémité supérieure dudit tube.

14. Appareil d'analyse comportant :
- un portoir de flacons (50) ;
- un support de récipients (60) ;
- un porte-doigts (70) comportant des doigts ;
- optionnellement, un portoir (81) de substrats d'analyse (80) ;
- un mécanisme (55) configuré pour :
- introduire les doigts (72) du porte-doigts dans des récipients (12) de dispositifs de capture selon l'une quelconque des revendications 1 à 11 respectifs débouchant chacun, à des extrémités inférieure et supérieure, par des ouvertures inférieure (26i) et supérieure (26s), respectivement, et disposés sur le support de récipients, de manière à constituer un portoir de récipients (52), chaque doigt (72) présentant une extrémité inférieure qui, après constitution du portoir de récipients, est en contact avec un ressort (30) d'un dit dispositif de capture de manière que ledit ressort soit en contact avec le bloc absorbant dudit dispositif de capture, ledit bloc absorbant soit en contact avec le tampon dudit dispositif de capture, et ledit tampon soit en contact avec la membrane filtrante dudit dispositif de capture,
- introduire lesdits récipients, disposés sur le support de récipients, dans des flacons (56) respectifs disposés sur le portoir de flacons ou pour extraire lesdits récipients hors desdits flacons respectifs, et
- optionnellement, appliquer les membranes filtrantes (14) desdits récipients sur des substrats d'analyse (80) respectifs, de préférence disposés sur le portoir de substrats d'analyse.

15. Appareil d'analyse selon la revendication immédiatement précédente, comportant un ensemble de soufflets (76), et dans lequel chaque doigt (72) est percé d'une lumière (74), ladite lumière débouchant par des ouvertures inférieure (74i) et supérieure (74s) en communication de fluide avec le volume intérieur d'un récipient respectif, après constitution du portoir de récipients, et avec le volume intérieur d'un soufflet respectif (76), respectivement.

16. Appareil d'analyse selon la revendication immédiatement précédente, comportant un vérin (78) configuré de manière à sélectivement presser ledit soufflet afin d'augmenter la pression à l'intérieur dudit récipient.

17. Procédé de préparation d'un échantillon destiné à une analyse biologique, en particulier cytologique, ledit procédé comportant les étapes suivantes :
a) obtention d'au moins un flacon (56) contenant un milieu liquide contenant des particules biologiques et disposition dudit flacon dans un portoir de flacons (50) d'un appareil d'analyse selon l'une quelconque des quatre revendications immédiatement précédentes ;
b) indépendamment de l'étape a), disposition d'un dispositif de capture (10) selon l'une quelconque des revendications 1 à 11, sur un support de récipients (60) dudit appareil d'analyse ;
c) de préférence, après l'étape b), introduction d'un doigt d'un porte-doigts (70) dudit appareil d'analyse, par une ouverture supérieure du récipient du dispositif de capture, le doigt étant conformé de manière que, dans la position d'introduction maximale du doigt dans le récipient, le tampon contenu dans le récipient soit en contact sur la membrane filtrante fixée sur ledit récipient ;
d) introduction de la membrane filtrante du récipient dans ledit flacon et maintien du récipient en position pendant une durée suffisante pour que le bloc absorbant du dispositif de capture absorbe dudit milieu liquide entrant à l'intérieur du récipient à travers la membrane filtrante du dispositif de capture ;
e) extraction du récipient hors du flacon ;
f) récupération de particules biologiques retenues sur la membrane filtrante par :
- maintien du récipient hors de flacon pendant une période d'attente supérieure à 5s, puis
- application de la membrane filtrante sur un substrat d'analyse (80) disposé sur un portoir de substrats d'analyse de l'appareil d'analyse, puis
- augmentation de la pression à l'intérieur du récipient de manière à créer un flux de milieu liquide sortant du récipient à travers la membrane filtrante.

## Patentansprüche

1. Vorrichtung zur Erfassung biologischer Partikel, die in einem flüssigen Medium suspendiert sind, wobei die Vorrichtung aufweist:
- einen Behälter (12), der über eine untere Öffnung nach außen mündet;
- eine Filtermembran (14), die so an dem Behälter befestigt ist, dass sie die untere Öffnung verschließt; und
im Inneren des Behälters:
- ein Puffer (16) aus porösem Schaumstoff, der eine ebene Fläche aufweist, die auf der Filtermembran ruht;
- ein Absorberblock (18), der auf dem Puffer ruht und geeignet ist, flüssiges Medium zu absorbieren, wenn er sich in Kontakt mit dem flüssigen Medium befindet; und
- eine Feder (30), die dazu eingerichtet ist, der Ausdehnung und/oder der Verlagerung des Absorberblocks von der unteren Öffnung des Behälters weg entgegenzuwirken.

2. Vorrichtung nach dem unmittelbar vorhergehenden Anspruch, wobei die entlang der Achse X gemessene Dicke des Puffers größer als 1 mm und kleiner als 4 mm ist.

3. Vorrichtung nach einem der vorhergehenden Ansprüche, wobei der Puffer aus Polyurethan besteht.

4. Vorrichtung nach einem der vorhergehenden Ansprüche, wobei der Puffer (16) eine Unterseite (16i) mit einer zur Oberseite (14s) der Filtermembran (14) komplementären Form aufweist.

5. Vorrichtung nach einem der vorhergehenden Ansprüche, wobei der Puffer (16) mit über 80 % einer Oberseite (14s) der Filtermembran (14) in Kontakt steht.

6. Vorrichtung nach einem der vorhergehenden Ansprüche, welche einen Anschlag (72) aufweist, welcher der Verlagerung der Feder von der unteren Öffnung des Behälters weg entgegenwirkt.

7. Vorrichtung nach einem der vorhergehenden Ansprüche, wobei die Feder ein Block aus elastischem Schaumstoff ist.

8. Vorrichtung nach einem der vorhergehenden Ansprüche, wobei der Schaumstoffblock derart ausgebildet ist, dass er in der Position, in welcher er im Inneren des Behälters aufgenommen ist, durch die Wand des Behälters zusammengedrückt wird.

9. Vorrichtung nach einem der vorhergehenden Ansprüche, wobei die mittlere Größe der Poren der Filtermembran größer als ein Mikrometer und/oder kleiner als 25 Mikrometer ist und/oder der Absorberblock aus einem hydrophilen Material besteht.

10. Vorrichtung nach einem der vorhergehenden Ansprüche, wobei der Behälter ein Rohr mit einer Achse X ist, das an einem unteren und an einem oberen Ende über eine untere (26i) bzw. obere Öffnung (26s) nach außen mündet, wobei die Feder geeignet ist, der Ausdehnung und/oder der Verlagerung des Absorberblocks zum oberen Ende des Rohres hin entgegenzuwirken.

11. Vorrichtung nach dem unmittelbar vorhergehenden Anspruch, wobei die Differenz zwischen den größten Querabmessungen der oberen Öffnung des Rohres einerseits und des Puffers andererseits größer als 0,2 mm und kleiner als 4 mm ist.

12. Verfahren zum Erfassen biologischer Partikel, die in einem flüssigen Medium suspendiert sind, mittels einer Vorrichtung zur Erfassung nach einem der vorhergehenden Ansprüche, wobei das Verfahren die folgenden Schritte umfasst:
i) Eintauchen der Filtermembran des Behälters der Vorrichtung zur Erfassung in das flüssige Medium;
ii) Halten des Behälters in dieser Position während einer Dauer, die ausreichend ist, um auf der Filtermembran der Vorrichtung zur Erfassung Partikel zurückzuhalten, die in einem Strom von flüssigem Medium enthalten sind, der in den Behälter eintritt und durch die Absorption des flüssigen Mediums durch den Absorberblock der Vorrichtung zur Erfassung erzeugt wird;
iii) Herausziehen des Behälters aus dem flüssigen Medium und, optional, Aufbringen der Filtermembran auf ein Analysesubstrat.

13. Verfahren nach dem unmittelbar vorhergehenden Anspruch, wobei der Behälter ein Rohr mit einer Achse X ist, das an einem unteren und an einem oberen Ende über eine untere (26i) bzw. obere Öffnung (26s) nach außen mündet, und wobei im Schritt i) das untere Ende Rohres in das flüssige Medium eingetaucht wird und dabei das obere Ende des Rohres so gehalten wird, dass es herausragt.

14. Analysegerät, welches aufweist:
- ein Bechergestell (50);
- einen Behälterträger (60);
- einen Fingerträger (70), der Finger aufweist;
- optional ein Gestell (81) für Analysesubstrate (80);
- einen Mechanismus (55), der dafür ausgelegt ist:
- die Finger (72) des Fingerträgers in Behälter (12) von jeweiligen Vorrichtungen zur Erfassung nach einem der Ansprüche 1 bis 11 einzuführen, die jeweils an einem unteren und an einem oberen Ende über eine untere (26i) bzw. obere Öffnung (26s) nach außen münden und auf dem Behälterträger angeordnet sind, so dass sie ein Behältergestell (52) bilden, wobei jeder Finger (72) ein unteres Ende aufweist, welches sich nach Bildung des Behältergestells in Kontakt mit einer Feder (30) einer besagten Vorrichtung zur Erfassung befindet, derart, dass die Feder sich in Kontakt mit dem Absorberblock der Vorrichtung zur Erfassung befindet, der Absorberblock sich in Kontakt mit dem Puffer der Vorrichtung zur Erfassung befindet und der Puffer sich in Kontakt mit der Filtermembran Vorrichtung zur Erfassung befindet,
- die Behälter, die auf dem Behälterträger angeordnet sind, in jeweilige Becher (56) einzuführen, die auf dem Bechergestell angeordnet sind, oder die Behälter aus den jeweiligen Bechern herauszuziehen, und
- optional die Filtermembranen (14) der Behälter auf jeweilige Analysesubstrate (80) aufzubringen, die vorzugsweise auf dem Gestell für Analysesubstrate angeordnet sind.

15. Analysegerät nach dem unmittelbar vorhergehenden Anspruch, welches eine Anordnung von Balgen (76) aufweist, und wobei jeder Finger (72) von einem Loch (74) durchquert wird, wobei das Loch über eine untere (74i) und eine obere (74s) Öffnung nach außen mündet, die mit dem Innenvolumen eines jeweiligen Behälters, nach Bildung des Behältergestells, bzw. mit dem Innenvolumen eines jeweiligen Balgs (76) in Fluidverbindung stehen.

16. Analysegerät nach dem unmittelbar vorhergehenden Anspruch, welches einen Zylinder (78) aufweist, der dafür ausgelegt ist, selektiv den Balg zu drücken, um den Druck im Inneren des Behälters zu erhöhen.

17. Verfahren zur Herstellung einer Probe, die für eine biologische, insbesondere zytologische, Analyse bestimmt ist, wobei das Verfahren die folgenden Schritte umfasst:
a) Erhalten mindestens eines Bechers (56), der ein flüssiges Medium enthält, das biologische Partikel enthält, und Anordnen des Bechers in einem Bechergestell (50) eines Analysegerätes nach einem der vier unmittelbar vorhergehenden Ansprüche;
b) unabhängig von Schritt a), Anordnen einer Vorrichtung zur Erfassung (10) nach einem der Ansprüche 1 bis 11 auf einem Behälterträger (60) des Analysegerätes;
c) vorzugsweise nach Schritt b), Einführen eines Fingers eines Fingerträgers (70) des Analysegerätes durch eine obere Öffnung des Behälters der Vorrichtung zur Erfassung, wobei der Finger derart ausgebildet ist, dass in der Position maximaler Einführung des Fingers in den Behälter der in dem Behälter enthaltene Puffer sich in Kontakt mit der an dem Behälter befestigten Filtermembran befindet;
d) Einführen der Filtermembran des Behälters in den Becher und Halten des Behälters in dieser Position während einer Dauer, die ausreichend dafür ist, dass der Absorberblock der Vorrichtung zur Erfassung flüssiges Medium absorbiert, das durch die Filtermembran der Vorrichtung zur Erfassung hindurch ins Innere des Behälters eintritt;
e) Herausziehen des Behälters aus dem Becher;
f) Sammeln von biologischen Partikeln, die auf der Filtermembran zurückgehalten wurden, durch:
- Halten des Behälters außerhalb des Bechers während einer Wartezeit, die länger als 5 s ist, danach
- Aufbringen der Filtermembran auf ein Analysesubstrat (80), das auf einem Gestell für Analysesubstrate des Analysegerätes angeordnet ist, danach
- Erhöhung des Druckes im Inneren des Behälters, um so einen Strom von flüssigem Medium zu erzeugen, der aus dem Behälter durch die Filtermembran hindurch austritt.

## Claims

1. Device for capturing biological particles in suspension in a liquid medium, the device having:
- a container (12) that is open via a lower opening;
- a filter membrane (14) fixed on the container in such a way as to close the lower opening; and
inside the container:
- a buffer (16) made of a porous foam and having a planar face resting on the filter membrane;
- an absorbent block (18) resting on the buffer and able to absorb said liquid medium when it is in contact with said liquid medium; and
- a spring (30) designed to impede the expansion and/or movement of the absorbent block away from the lower opening of the container.

2. Device according to the immediately preceding claim, in which the thickness of the buffer, measured along the axis X, is greater than 1 mm and less than 4 mm.

3. Device according to either of the preceding claims, in which the buffer is made of polyurethane.

4. Device according to any one of the preceding claims, in which the buffer (16) has a lower face (16i) with a shape complementary to the upper face (14s) of the filter membrane (14).

5. Device according to any one of the preceding claims, in which the buffer (16) is in contact with more than 80% of an upper face (14s) of the filter membrane (14).

6. Device according to any one of the preceding claims, having a limit stop (72) impeding the movement of the spring away from the lower opening of the container.

7. Device according to any one of the preceding claims, in which the spring is a block of elastic foam.

8. Device according to any one of the preceding claims, in which the foam block is shaped in such a way that, in a position in which it is housed inside the container, it is compressed by the wall of the container.

9. Device according to any one of the preceding claims, in which the average pore size of the filter membrane is greater than one micron and/or less than 25 microns, and/or the absorbent block is made of a hydrophilic material.

10. Device according to any one of the preceding claims, in which the container is a tube of axis X opening out at lower and upper ends via lower (26i) and upper (26s) openings, respectively, the spring being able to impede the expansion and/or movement of the absorbent block toward the upper end of the tube.

11. Device according to the immediately preceding claim, in which the difference between the greatest transverse dimensions of the upper opening of the tube, on the one hand, and of the buffer, on the other hand, is greater than 0.2 mm and less than 4 mm.

12. Method for capturing biological particles in suspension in a liquid medium, by means of a capture device according to any one of the preceding claims, said method having the following steps:
i) immersing the filter membrane of the container of said capture device in the liquid medium;
ii) maintaining the container in position for a duration that is sufficient to retain, on the filter membrane of said capture device, particles contained in a flow of liquid medium entering the container and generated by the absorption of said liquid medium by the absorbent block of said capture device;
iii) withdrawing the container from the liquid medium and, optionally, applying the filter membrane to an analysis substrate.

13. Method according to the immediately preceding claim, in which the container is a tube of axis X, opening out at lower and upper ends via lower (26i) and upper (26s) openings, respectively, and in which, in step i), the lower end of the tube is immersed in the liquid medium, the upper end of said tube being kept above the surface of the liquid medium.

14. Analysis apparatus having:
- a vial rack (50);
- a container support (60);
- a finger holder (70) having fingers;
- optionally, a rack (81) of analysis substrates (80);
- a mechanism (55) configured to:
- introduce the fingers (72) of the finger holder into respective containers (12) of capture devices according to any one of Claims 1 to 11, each of these opening out, at lower and upper ends, via lower (26i) and upper (26s) openings, respectively, and being arranged on the container support, in such a way as to constitute a container rack (52), each finger (72) having a lower end which, after formation of the container rack, is in contact with a spring (30) of a said capture device, in such a way that said spring is in contact with the absorbent block of said capture device, said absorbent block is in contact with the buffer of said capture device, and said buffer is in contact with the filter membrane of said capture device,
- introduce said containers, arranged on the container support, into respectve vials (56) arranged on the vial rack, or withdraw said containers from said respective vials, and,
- optionally, apply the filter membranes (14) of said containers to respective analysis substrates (80), preferably arranged on the analysis substrate rack.

15. Analysis apparatus according to the immediately preceding claim, having a set of bellows (76), and in which each finger (72) is pierced with a lumen (74), said lumen opening out via lower (74i) and upper (74s) openings that are in fluidic communication respectively with the internal volume of a respective container, after formation of the container rack, and with the internal volume of a respective bellows (76).

16. Analysis apparatus according to the immediately preceding claim, having an actuator (78) configured in such a way as to selectively press said bellows in order to increase the pressure inside said container.

17. Method for preparing a sample intended for biological analysis, in particular cytological analysis, said method having the following steps:
a) obtaining at least one vial (56) containing a liquid medium containing biological particles, and arranging said vial in a vial rack (50) of an analysis apparatus according to any one of the four immediately preceding claims;
b) independently of step a), arranging a capture device (10) according to any one of Claims 1 to 11 on a container support (60) of said analysis apparatus;
c) preferably, after step b), introducing a finger of a finger holder (70) of said analysis apparatus through an upper opening of the container of the capture device, the finger being configured in such a way that, in the position of maximum introduction of the finger into the container, the buffer contained in the container is in contact with the filter membrane fixed on said container;
d) introducing the filter membrane of the container into said vial and maintaining the container in position for a duration that is sufficient for the absorbent block of the capture device to absorb said liquid medium entering the interior of the container through the filter membrane of the capture device;
e) withdrawing the container from the vial;
f) recovering biological particles retained on the filter membrane by:
- holding the container out of the vial for a waiting period of more than 5 seconds, then
- applying the filter membrane to an analysis substrate (80) arranged on an analysis substrate rack of the analysis apparatus, then
- increasing the pressure inside the container in such a way as to create a flow of liquid medium exiting the container through the filter membrane.
